# EUROPEAN PATENT SPECIFICATION

(11) **EP 1 226 798 B1**
(45) Date of publication and mention of the grant of the patent: **04.10.2006**
(21) Application number: 01307148.5
(22) Date of filing: 22.08.2001
(51) Int. Cl.: A61F 2/06

(54) **Mounting method for low profile balloon expandable stent**
Montageverfahren für ballonexpandierende Stents mit geringem Querschnitt
Procédé de montage d'endoprothèses de faible diamètre déployables par ballonnet

(30) Priority: 23.08.2000 US 644082
(43) Date of publication of application: 31.07.2002
(73) Proprietor: Cordis Corporation, Miami Lakes, Florida 33014 (US)
(72) Inventor: Lorenzo, Juan A., Fort Lauderdale, Florida 33328 (US); Fischell, David R., Fair Haven, New Jersey 07704 (US)
(74) Representative: Fisher, Adrian John

(56) References cited:
- WO-A-97/07756
- US-A- 5 490 839
- US-A- 5 836 965
- US-A- 6 018 857

## Description

### FIELD OF USE

This invention is in the field of balloon expandable stents that are used to maintain patency of a vessel of the human body.

### BACKGROUND OF THE INVENTION

It has been shown that intravascular stents are an excellent means to maintain the patency of blood vessels following balloon angioplasty. US patent No. 6190403 , filed November 13, 1998, Fischell *et*. *al*, describes a flexible low profile stent design with N, inverted N, M or W shaped undulating longitudinal members connecting the circumferential strut members. During stent delivery, these undulating longitudinal members now incorporated into the CORDIS BX Velocity ® stent, can lengthen and shorten as the stent is pushed around a bend providing a means for the stent to bend easily.

Most balloon expandable stents are mounted on the balloon by first sliding the cut stent over the balloon and aligning the ends of the stent, with the radio-opaque marker bands manually. The stent is then crimped down with pressure. This by itself will push the balloon material up into the interstices of the stent. To provide even more secure mounting, a second process to nest the stent is now in use where the crimped stent is placed inside a tube and the balloon is pressurized, to mold the balloon material further up into the interstices of the stent.

The prior art crimping process has the following properties:
1. Cut stents have some variation in their length as to the marker band placements on the balloon shaft. Furthermore, the edges of the balloon have some variation in location with respect to the marker bands. As a result it is difficult to accurately match the end of the stent to the transition edge of the balloon. (The distance between the end of the stent and the balloon transition is called the balloon overhang.) It is nearly impossible with current techniques to get the balloon overhang to less than 1 mm on each side of the stent. It is highly desirable to reduce balloon overhang to as much as possible to avoid edge dissections and injury beyond the end of the stent.
2. When the nest process is performed on the previously crimped stent, the stent on the balloon is slid into a nesting tube that typically has an inside diameter as much as 0.002" (0.05 mm) larger than the outside diameter of the crimped stent. Thus, when the stent is nested within the nesting tube, there can be as much as a 0.002" increase in system diameter (stent profile) from the pre-nesting device.

Another method is disclosed in US 6018857, in which the operation is performed normally, the crimping involving rolling the assembled device between a person's fingers.

### SUMMARY OF THE INVENTION

The present invention provides a balloon expandable stent delivery catheter as defined in claim 1. The features of the catheter known from US 6018857 have been placed in the preamble of claim 1.

This invention allows an improvement of the process for mounting stents on a balloon stent delivery catheter. The first improvement involves a process for eliminating balloon overhang beyond the ends of the stent. This process is particularly useful with undulating longitudinal stents such as those described in U.S. patent 5,879,370, assigned to a common assignee. Undulating longitudinal stents use flexible links to connect the circumferential struts. The flexible links are adapted to lengthen and shorten as the stent is advanced around a curve allowing the stent to easily bend. The ability of the flex links to lengthen and shorten is also of significance to the present invention process for reducing balloon overhang.

The first step of the process occurs during manufacturing of the stent delivery balloon. In accordance with the invention, an edge of the cylindrical portion of the balloon can be marked to allow exact adjustment of the stent location and length to eliminate balloon overhang. The present inventions marking can be a thin marker line in the balloon at the edge, a line drawn around the balloon at the edge, a modified surface finish of the entire balloon outside of the edges or any other marking that would allow the technician mounting the stent to visualize the balloon edges. A well-performed balloon folding process will maintain the position of the edge mark. Alternately, one could mark the central cylinder of the balloon by a line extending the length of the cylinder, a difference in surface texture or any other set of marks. The marking, however, is by way of send blasting to change the surface of the balloon in the area of said marks.

Once the marked balloon has been attached to the catheter shaft and then folded, the second mounting step of the stent onto the balloon can begin. The apparatus for stent mounting has the capability to grab the end struts of the stent. Once the stent has been grabbed, the apparatus allows the stent to be precisely moved longitudinally with respect to the balloon. The apparatus then can be used to stretch the stent by holding one end fixed and pulling in the opposite direction on the other grabbed stent end. The stent can now be crimped in place with the stent ends exactly matched to the balloon edges thus eliminating balloon overhang. Typically, the stent would start at a length 0.5 to 2.0 mm shorter than the balloon and be stretched to exactly match the length of the balloon cylindrical section.

The final step in the process is to nest the stent onto the balloon. Unlike the prior art process where the stent is simply placed in a tube and the balloon is lightly expanded, the present invention uses pressure applied outside the nesting tube to prevent the stent from expanding outward during the nesting process. This process is called compression nesting. Ideally, the same apparatus used for crimping can be heated and used for nesting the stent as well. The preferred embodiment of this step has the mounted stent slid into a standard crimping tube before it is compression nested.

It is also envisioned that the inward pressure from the crimping apparatus would be sufficient to cause the stent to become nested into the balloon without the need for pressurizing the balloon. This process can be further improved by closing off the balloon inflation lumen before pressure is applied. This will cause an increased pressure inside the balloon as the stent is squeezed onto the balloon. An additional improvement would place a wire within the balloon lumen to reduce the volume of the closed space before sealing of the balloon lumen. A luer cap with a wire in its center would be ideal for this purpose. Eliminating the use of balloon inflation during nesting will reduce the time and cost of manufacturing the product.

Thus it is an object of this invention to enable a process for mounting a stent on an angioplasty balloon with no balloon overhang.

Another object of this invention is to enable a compression nesting process for nesting a stent onto a balloon that prevents the outward expansion of the stent during the low-pressure balloon inflation step.

Still another object of this invention is to enable a process for nesting a stent using only pressure from the outside of the stent.

It is desirable to seal the balloon lumen at its proximal end before compression nesting.

It is derivable to place a wire down the balloon lumen to reduce the volume inside the balloon before compression nesting.

It is desirable to have a luer cap with a wire through the center to both seal the balloon lumen and to reduce the balloon lumen volume during compression nesting.

These and other objects and advantages of this invention will become obvious to a person of ordinary skill in this art upon reading of the detailed description of this invention including the associated drawings.

### BRIEF DESCRIPTION OF THE DRAWINGS

FIG. 1 is a sketch of an apparatus for grit blasting the transition sections of a molded angioplasty balloon after molding.
FIG. 2 is a sketch of a balloon expandable stent delivery catheter mold with grit blasted end sections to change the surface finish of the balloon catheter's ends.
FIG. 3 is a side view of the balloon catheter with grit blasted transition sections in accordance with the invention.
FIG. 4 is a sketch of an apparatus for locating and stretching a stent before crimping onto a balloon.
FIG. 5 is a flat layout of a stent appropriate for use with the apparatus of FIG. 4.
FIG. 6 is a sketch showing the apparatus of FIG. 4 in use for locating and stretching a stent before crimping onto a balloon.
FIG. 7A is a longitudinal cross section of a stent crimped on a balloon prior to the nesting process.
FIG. 7B is a longitudinal cross section of the prior art apparatus for nesting a stent onto a balloon.
FIG. 8 is a longitudinal cross section of an apparatus for compression nesting a stent onto a balloon.
FIG. 9 is a longitudinal cross section of the proximal end of a balloon catheter fitted with a luer cap having a wire through its center so as to reduce the volume of the balloon lumen during compression nesting.

### DETAILED DESCRIPTION OF THE DRAWINGS

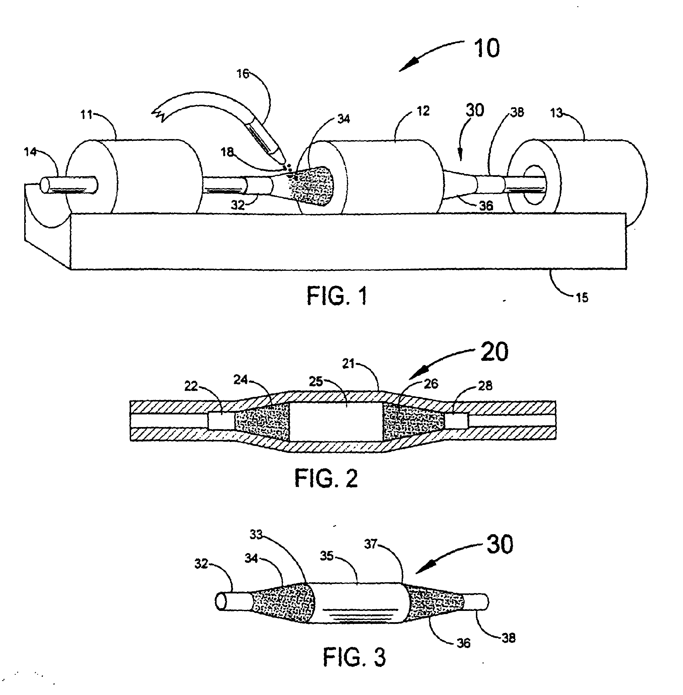

FIG. 1 is a sketch of an apparatus 10 for grit blasting the transition sections 34 and 36 of a molded angioplasty balloon 30 after molding. The apparatus 10 includes a mold base 15, proximal mold section 11, central mold section 12 and distal mold section 13. A central mandrel 14 runs down the center of the three mold sections, 11, 12 and 13 and forms a means to open the apparatus 10 after the balloon 30 has been molded. The balloon 30 also has proximal shaft 32, and distal shaft 38 that are attached to the balloon catheter during assembly. The molding and assembly of balloon angioplasty catheters is well known. After the balloon 30 has been molded, and the proximal and distal mold sections 11 and 13 respectively have been slid away from the central mold section 12, a grit blasting nozzle 16 can be used to change the surface finish of the proximal and distal balloon transition sections 34 and 36 respectively, by firing grit particles 18 at the balloon surface. To most easily accomplish the preceding grit blasting process, the balloon 30 should be partly or completely inflated.

FIG. 2 is a sketch of a balloon mold 20 having mold cavity 21 with grit blasted proximal and distal mold transition sections 24 and 26 respectively. The mold cavity 21 also has proximal shaft section 22, central section 25 and distal shaft 28. During balloon blowing, the grit blasted pattern is transferred to the balloon surface. By grit blasting only the sections 24 and 26 of the mold cavity 21, the molded balloon 30, shown in FIG. 3, will have an altered surface finish of the balloon transition sections 34 and 36 where the balloon material touches the grit blasted mold sections 24 and 26.

FIG. 3 is a side view of the balloon 30 with transition sections 34 and 36 having an altered surface finish as compared to the central cylindrical section 35. The balloon 30 also has proximal shaft 32, and distal shaft 38 that are attached to the balloon catheter during assembly. The central section 35 has proximal and distal edges 33 and 37.

The altered surface finish of the proximal and distal transition sections 34 and 36 respectively serve as marks to facilitate visualization of the distal and proximal edges 33 and 37.

It is also envisioned that one or more marks placed at the proximal and distal edges 33 and 37 respectively could also serve to facilitate visualization of the distal and proximal edges 33 and 37. These marks could be applied to the balloon after molding or during the molding process. Examples of such marks are a single circumferential line placed at the location of the balloon edge, a series of dots placed around the balloon at the location of the edges, a series of short longitudinal lines placed around the balloon at the location of the edges.

It is also envisioned that the central section 35 could have altered surface appearance and the proximal and distal transition sections 34 and 36 have a normal surface appearance.

It is also envisioned that the central section of the balloon could be tapered rather than cylindrical to facilitate introduction of a stent into a tapered vessel.

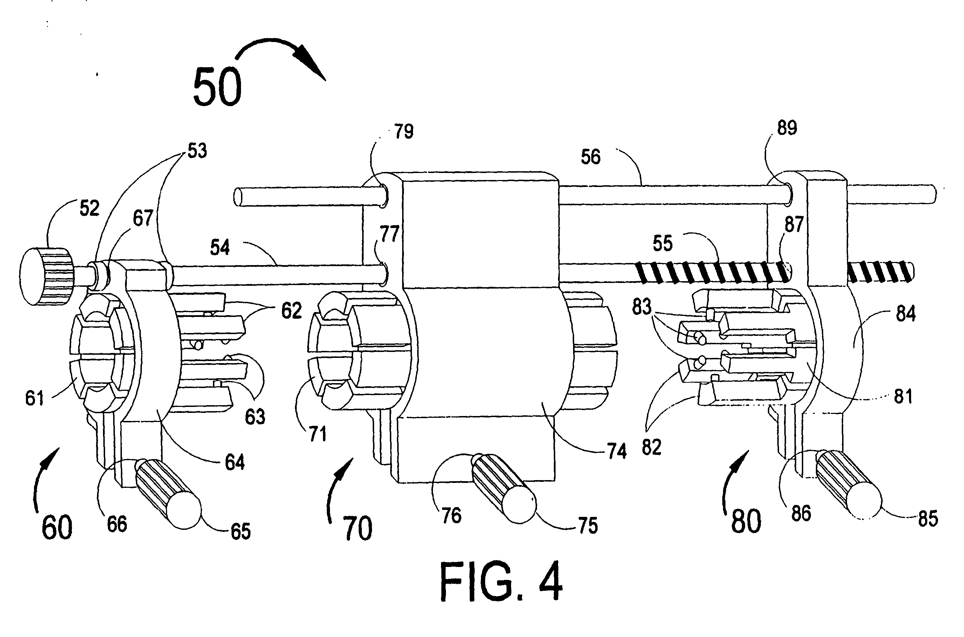

FIG. 4 is a sketch of the apparatus 50 for locating and stretching a stent before crimping onto a stent delivery balloon such as the balloon 30 shown in FIG. 3. The apparatus 50 has three sections, a distal grabber 60, a proximal grabber 80 and a central crimping tool 70.

The distal grabber 60 has an outer housing 64 and a collet 61 with 6 pieces having arms 62 with each arm having a catch pin 63. The catch pins 63 will engage the most distal strut of a stent to be mounted. A threaded rod 66 with handle 65 is used to compress the collet 61 moving the arms 62 with catch pins 63 radially inward.

The proximal grabber 80 has an outer housing 84 a collet 81 with 6 pieces having arms 82 with each arm having a catch pin 83. The catch pins 83 will engage the most distal strut of a stent to be mounted. A threaded rod 86 with handle 85 is used to compress the collet 81 moving the arms 82 with catch pins 83 radially inward.

The central crimping tool 70 has an outer housing 74 that is used to compress the crimping collet 71 when the operator turns the handle 75 attached to the threaded rod 76.

A guide rod 56 runs through the hole 79 in the top of the outer housing 74 of the central crimping tool 70 and the hole 89 in the top of the outer housing 84 of the proximal grabber 80.

A stent length adjustment rod 54 having distal threads 55, a proximal handle 52 and stops 53 passes through the hole 67 in the top of the outer housing 64 of the distal grabber 60, the hole 77 in the outer housing 74 of the central crimping tool 70 and the threaded hole 87 in the outer housing 84 of the proximal grabber 80.

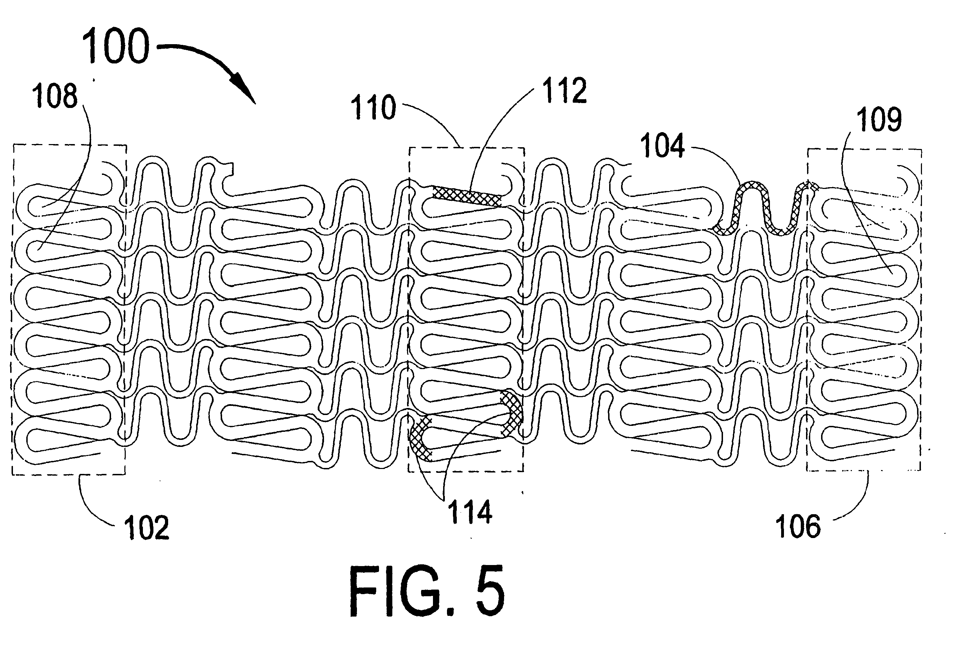

FIG. 5 is a flat layout of a stent 100 having struts 110 with diagonals 112 and connecting arcs 114. The stent struts 110 are the circumferential elements that open up as the stent expands. The struts 110 are connected one to the other by flex segments 104 attached to the connecting arcs 114. The stent has a most distal strut 102 and a most proximal strut 106. The catch pins 63 of the distal grabber 60 of FIG. 4 will fit into the interstices 108 inside the most distal connecting arcs 114 of the distal strut 102. The catch pins 83 of the proximal grabber 80 of FIG. 4 will fit into the interstices 109 inside the most proximal connecting arcs 114 of the proximal strut 106.

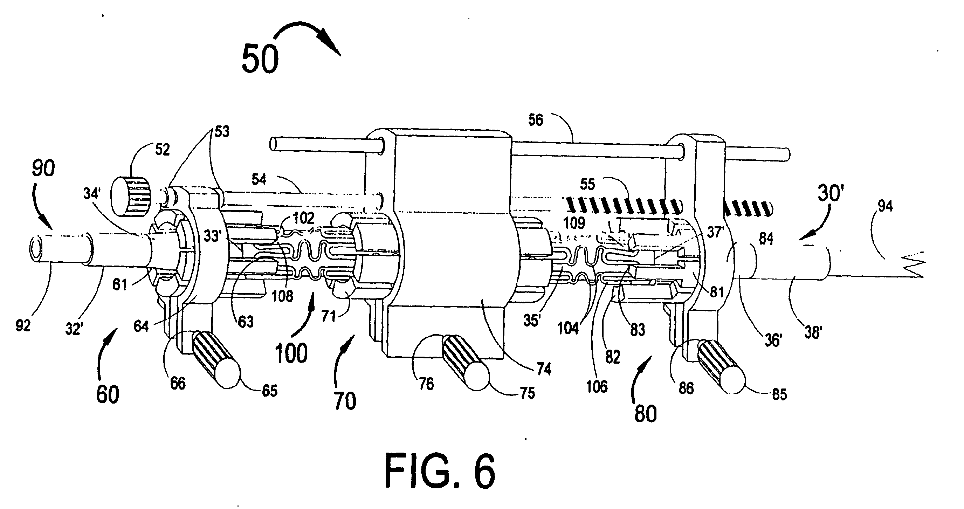

FIG. 6 shows the apparatus 50 in use during the mounting of a stent 100 on a stent delivery catheter 90 having folded balloon 30'. The stent delivery catheter 90 has inner shaft 92 with guide wire lumen 96 and outer shaft 94. The stent delivery catheter 90 can be either an over-the-wire or rapid exchange type catheter. The folded balloon 30' has transition sections 34' and 36' having an altered surface finish as compared to the central cylindrical section 35'. The folded balloon 30' also has proximal shaft 32' attached to the outer shaft 94 and a distal shaft 38' attached to the inner shaft 92 of the stent delivery catheter 90 during assembly. The central section 35' has proximal and distal edges 33' and 37'.

The stent 100, also shown in flat layout view in FIG. 5, has distal strut 102, proximal strut 104 and "N" shaped flex segments 104.

The method for using the apparatus 50 to mount the stent 100 with no balloon overhang has the following steps:
1. Slide the stent 100 onto the folded balloon 30' of the stent delivery catheter 90.
2. Open the collets 61, 71 and 81 to a diameter larger than that of the stent 100.
3. Slide the stent delivery catheter 90 through the three collets 81, 71, and 61 and rotate the catheter 90 as necessary until the proximal interstices 109 in the most proximal strut 106 are aligned with the catch pins 83 of the proximal grabber 80.
4. Tighten the collet 81 by turning the handle 85 attached to the threaded rod 86 until the catch pins 83 are inserted into the interstices 109.
5. Tighten or loosen the threaded rode 54 by turning the handle 52 until the catch pins 63 of the distal grabber 60 are aligned with the interstices 108 of the most distal strut 108.
6. Tighten the collet 61 by turning the handle 65 attached to the threaded rod 86 until the catch pins 63 are inserted into the interstices 109. This is the exact stage of the apparatus 50 as shown in FIG. 6.
7. Push the stent delivery catheter 90 in a distal direction until the proximal edge of the proximal strut 106 of the stent 100 is lined up with the proximal balloon edge 37'. Further tighten the collet 81 by turning the handle 85 attached to the threaded rod 86 until the collet firmly holds onto the folded balloon 30' of the stent delivery catheter 90. It should be noted that the modified surface finish of the proximal transition section 36' allows the proximal balloon edge 37' to be clearly seen.
8. Once the proximal strut 106 is aligned and squeezed down onto the folded balloon 30', turn the handle 52 to rotate the threaded rod 54 thus pushing apart the distal grabber 60 from the proximal grabber 80. This will stretch the stent 100 by causing the flex segments 104 to unbend.
9. When the stent 100 has been stretched enough so that the distal end of the most distal strut 102 is aligned with the distal balloon edge 33', further tighten the collet 61 by turning the handle 65 attached to the threaded rod 66 until the collet firmly holds onto the folded balloon 30' of the stent delivery catheter 90.
10.Tighten the collet 71 of the central crimping tool 70 until the central section of the stent is squeezed down upon the folded balloon 30' of the stent delivery catheter 90. At this point, the stent 100 has been mounted onto the balloon and is exactly matched in length with the central section 35' of the folded balloon 30'
11. Loosen the collets 61, 71 and 81 and slide the stent delivery catheter 90 with mounted stent 100 out of the apparatus 50.

If the above process is the only process for crimping the stent 100 onto the folded balloon 30', then it is envisioned that a longer central crimping tool 70 would be used or the current tool 70 would be pushed against the proximal grabber 80. As the stent 100 is squeezed, then the central crimping tool 70 could be opened, slid against the distal grabber and a second squeeze performed.

It is also envisioned that after the above process, the stent delivery catheter 90 with stent 100 could be inserted into a standard crimping tube and the entire length of the stent 100 could be squeeze down uniformly.

The preferred method of mounting, however, would include an additional nesting process as follows.

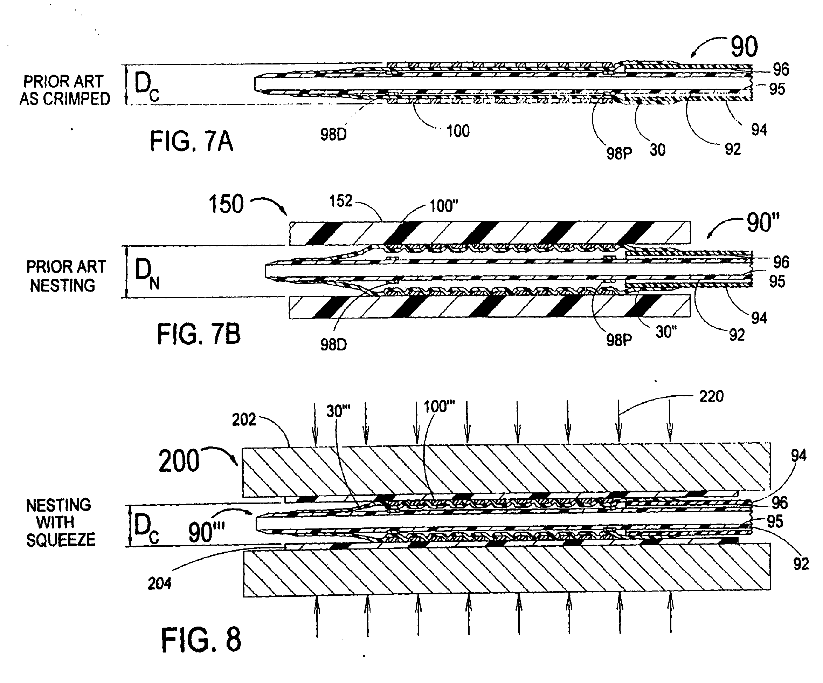

FIG. 7A is a longitudinal cross section of a stent 100 crimped on the balloon 30 of a stent delivery catheter 90 prior to the nesting process. The catheter 90 has distal marker band 98D, proximal marker band 98P, inner shaft 95, outer shaft 96 and balloon 30. It should be noted that the diameter of the stent 100 in FIG. 7A is D_{c}.

FIG. 7B is a longitudinal cross section of the prior art apparatus 150 after its use for nesting the stent 100" onto the balloon 30" of the stent delivery catheter 90". During nesting with the apparatus 150, the crimped stent 100 on delivery catheter 90 of FIG 7A is inserted into the tube 152, which may have a temperature higher than room temperature (although this is not always necessary.) The balloon 30" is then inflated with a gas such as nitrogen or carbon dioxide. This will push the balloon 30" and stent 100" outward against the inside of the nesting tube 152 which will cause the balloon 30" to mold into the interstices in the stent 100". The delivery catheter 90" with nested stent is then removed from the nesting tube 152 to cool. The resulting nested stent 100" will have a diameter D_{N} that is typically 0.004 inches larger than the crimped diameter D_{C} of FIG. 7A.

FIG. 8 is a longitudinal cross section of an apparatus 200 for nesting the stent 100''' onto the balloon 30''' of the stent delivery catheter 90'''. This technique can replace the standard stent crimping process (i.e., the stent 100 and catheter 90 shown in FIG. 7A) or can be used in conjunction with the modified stent mounting process done using the apparatus 50 shown in FIG. 6. In either case, the stent 100 on balloon 30 is placed in a crimping tube 204. The crimping tube is slid into a crimping collet 202, which again may have an elevated temperature. Pressure 220 is then applied to the crimping collet to compress the crimping tube 204 and stent 100''' diametrically inward. This compression nesting technique will simultaneously pressurize the balloon 30''' and compress the stent 100''' causing the balloon 30'' to become permanently molded into the interstices of the stent 100'''. Although additional pressurization of the balloon 30''' can be used in conjunction with compression nesting to further pressurize balloon 30''', it may only provide slight improvement of the nest while adding time and expense to the process.

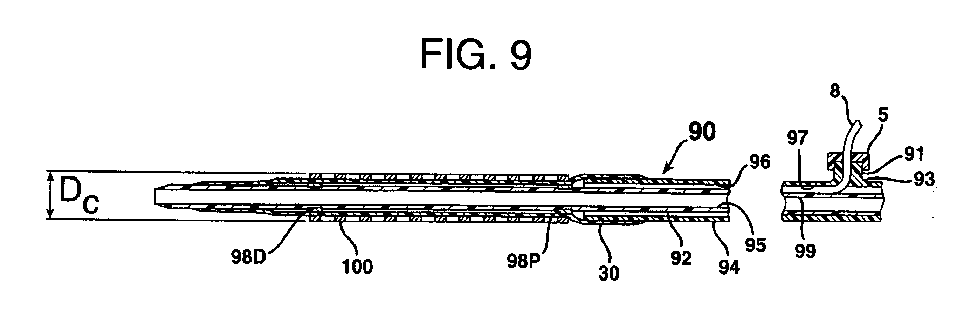

To facilitate the compression nesting without additional balloon pressurization a luer cap can be placed over the luer fitting typically attached to the proximal end of the balloon inflation lumen. A further improvement of this is shown in FIG. 9, which shows a longitudinal cross section of the proximal end of a balloon stent delivery catheter 90. The modified luer cap 5 with central wire 8 is shown attached to the proximal luer fitting 91. The standard guide wire Luer fitting 93 providing access to the guide wire lumen 95 is also shown. In FIG. 9 the central wire 8 is shown protruding down the balloon inflation lumen 97 of a dual lumen proximal shaft 99 of the stent delivery system 90. The balloon inflation lumen 97 is in fluid communication with the balloon inflation lumen 96 shown in FIG. 7A of the distal coaxial section of the stent delivery system 90. Techniques for attaching dual lumen balloon shafts to coaxial lumen balloon shafts are well known and have been explicitly shown in FIG. 7 of US Patent 5,910,145 to Fischell *et al*.. By placing the central wire down most of the length of the balloon inflation lumen 97, the pressure of the remaining air under the balloon 30''' of FIG. 8 will be higher, thus providing additional force to push and mold the plastic of the balloon 30''' into the interstices of the stent 100'''.

Although a luer cap has been described herein, any means for sealing the balloon inflation lumen at any point proximal to the balloon would accomplish the same result. For example a clamp placed on the catheter shaft 99 to squeeze closed the lumen or a plug inserted into the luer fitting 91. In addition, means other than a wire can be used to reduce the volume inside the balloon inflation lumen 97, for example the lumen 97 could be filled with a liquid such sterile water and then a simple Luer cap attached to the fitting 91.

After compression nesting the delivery catheter 90''' with nested stent is then removed from the collet 200 and crimping tube 204. Because the stent 100''' is being squeezed inward during the inflation of the balloon 30''', it will not expand outward like the stent 100" of FIG. 7B. As a result, the final diameter of the stent 100''' will be the same or less than the original crimped diameter D_{C}. This is the major advantage of using the apparatus 200. In other words, for the same balloon and stent configuration, the final device can have a significantly lower profile (diameter). This will enhance the deliverability of the product into vessels of the human body.

Various other modifications, adaptations, and alternative designs are of course possible in light of the above teachings. Therefore, it should be understood at this time that within the scope of the appended claims the invention may be practiced otherwise than as specifically described herein.

## Claims

1. A balloon expandable stent delivery catheter comprising proximal and distal ends and a molded balloon (30) attached to said catheter near said distal end of the catheter, the balloon (30) having a surface including a central stent delivery section (35) with proximal and distal edges (33, 37), **characterised by** the balloon surface further having radiopaque grit blasting marks (34, 36) thereon to facilitate visualization of both the proximal and distal edges (33, 37), and wherein said marks (34, 36) thereon are formed by means of grit blasting of the balloon (30) after the balloon has been molded so as to change the surface of the balloon (30) in the area of said marks (34, 36).

2. The catheter of claim 1 wherein the said marks (34, 36) are applied to the balloon (30) after the balloon (30) has been molded.

3. The catheter of claim 1 wherein the said marks (34, 36) are applied to the balloon (30) during the molding of the balloon (30).

4. The catheter of claim 1 wherein the said marks (34, 36) are lines placed around the balloon (30) at said proximal and distal edges (33, 37).

5. The catheter of claim 1 wherein the balloon (30) further comprises proximal and distal transition sections (32, 38), said marks (34, 36) being applied to alter the appearance of the distal and proximal transition sections (32, 38).

6. The catheter of claim 5 wherein the altered surface appearance is the result of grit blasting of the balloon transition sections after the balloon (30) has been molded.

7. The catheter of claim 5 wherein the altered surface appearance occurs as a result of the balloon molding process.

8. The catheter of claim 1 wherein the said marks (34, 36) are an altered surface appearance of the central section (35) of the balloon (30).

## Patentansprüche

1. Zuführungskatheter für ballonexpandierbare Stents mit einem proximalen und einem distalen Ende und einem geformten Ballon (30), der an dem Katheter nahe dem distalen Ende des Katheters angebracht ist, wobei der Ballon (30) eine Oberfläche aufweist, die einen mittleren Stentzuführungsabschnitt (35) mit einem proximalen und einem distalen Rand (33, 37) aufweist, **dadurch gekennzeichnet, daß** die Ballonoberfläche weiterhin röntgenstrahlundurchlässige Sandstrahlmarkierungen (34, 36) darauf aufweist, um der Sichtbarmachung von sowohl dem proximalen als auch dem distalen Rand (33, 37) zu unterstützen, und wobei die Markierungen (34, 36) darauf mittels Sandstrahlen des Ballons (30) gebildet werden, nachdem der Ballon geformt wurde, um die Oberfläche des Ballons (30) in dem Bereich der Markierungen (34, 36) zu ändern.

2. Katheter nach Anspruch 1, bei dem die Markierungen (34, 36) auf den Ballon (30) aufgebracht werden, nachdem der Ballon (30) geformt wurde.

3. Katheter nach Anspruch 1, bei dem die Markierungen (34, 36) auf den Ballon (30) während des Formens des Ballons (30) aufgebracht werden.

4. Katheter nach Anspruch 1, bei dem die Markierungen (34, 36) Linien sind, die um den Ballon (30) an dem proximalen und dem distalen Rand (33, 37) angeordnet sind.

5. Katheter nach Anspruch 1, bei dem der Ballon (30) weiterhin einen proximalen und einem distalen Übergangsabschnitt (32, 38) aufweist, wobei die Markierungen (34, 36) aufgebracht werden, um das Erscheinungsbild des distalen und des proximalen Übergangsabschnitts (32, 38) zu verändern.

6. Katheter nach Anspruch 5, bei dem das veränderte Oberflächenerscheinungsbild das Ergebnis des Sandstrahlens der Ballonübergangsabschnitte ist, nachdem der Ballon (30) gebildet wurde.

7. Katheter nach Anspruch 5, bei dem das geänderte Oberflächenerscheinungsbild als Ergebnis des Ballonformungsvorgangs auftritt.

8. Katheter nach Anspruch 1, bei dem die Markierungen (34, 36) ein verändertes Oberflächenerscheinungsbild des zentralen Abschnitts (35) des Ballons (30) sind.

## Revendications

1. Cathéter d'amenée d'endoprothèses déployables par ballonnet comprenant des extrémités proximale et distale et un ballonnet moulé (30) attaché audit cathéter près de ladite extrémité distale du cathéter, le ballonnet (30) ayant une surface comprenant une section centrale d'amenée d'endoprothèse (35) avec des bords proximal et distal (33, 37), **caractérisé en ce que** la surface du ballonnet comporte, en outre, des marques de grenaillage radio-opaques (34, 36) pour faciliter la visualisation des bords proximal et distal (33, 37) et dans lequel lesdites marques (34, 36) sont formées par grenaillage du ballonnet (30) une fois que le ballonnet a été moulé afin de modifier la surface du ballonnet (30) dans la zone desdites marques (34, 36).

2. Cathéter selon la revendication 1, dans lequel lesdites marques (34, 36) sont appliquées au ballonnet (30) une fois que le ballonnet (30) a été moulé.

3. Cathéter selon la revendication 1, dans lequel lesdites marques (34, 36) sont appliquées au ballonnet (30) pendant le moulage du ballonnet (30).

4. Cathéter selon la revendication 1, dans lequel lesdites marques (34, 36) sont des lignes placées autour du ballonnet (30) au niveau desdits bords proximal et distal (33, 37).

5. Cathéter selon la revendication 1, dans lequel le ballonnet (30) comprend, en outre, des sections proximale et distale de transition (32, 38), lesdites marques (34, 36) étant appliquées pour modifier l'aspect des sections proximale et distale de transition (32, 38).

6. Cathéter selon la revendication 5, dans lequel l'aspect de surface modifié est le résultat du grenaillage des sections de transition du ballonnet une fois que le ballonnet (30) a été moulé.

7. Cathéter selon la revendication 5, dans lequel l'aspect de surface modifié est le résultat du processus de moulage du ballonnet.

8. Cathéter selon la revendication 1, dans lequel lesdites marques (34, 36) sont un aspect de surface modifié de la section centrale (35) du ballonnet (30).
